(19) Europäisches Patentamt

European Patent Office

Office européen des brevets

(11) EP 0 508 472 B1

(12) EUROPEAN PATENT SPECIFICATION

(45) Date of publication and mention
of the grant of the patent:
**09.07.2003 Bulletin 2003/28**

(51) Int Cl.[7]: **C12N 5/16**, C07K 16/30,
A61K 39/395

(21) Application number: **92106282.4**

(22) Date of filing: **10.04.1992**

(54) **Method of producing hybridomas**

Verfahren zur Herstellung von Hybridomen

Procédé pour préparer des hybridomes

(84) Designated Contracting States:
**DE FR GB IT**

(30) Priority: **10.04.1991 JP 7751891**

(43) Date of publication of application:
**14.10.1992 Bulletin 1992/42**

(73) Proprietor: **KYOWA HAKKO KOGYO CO., LTD.**
**Chiyoda-ku, Tokyo-to (JP)**

(72) Inventors:
 • **Fujiwara, Katsumi**
   **Machida-shi, Tokyo (JP)**
 • **Shitara, Kenya**
   **Machida-shi, Tokyo (JP)**
 • **Ohta, So**
   **Machida-shi, Tokyo (JP)**
 • **Hanai, Nobuo**
   **Sagamihara-shi, Kanagawa (JP)**

(74) Representative: **Kinzebach, Werner, Dr. et al**
**Patentanwälte**
**Reitstötter, Kinzebach und Partner**
**Postfach 86 06 49**
**81633 München (DE)**

(56) References cited:
**EP-A- 0 369 425        WO-A-90/02795**
**WO-A-91/17769        US-A- 4 444 887**

 • JOURNAL EXPERIMENTAL MEDICINE vol. 155,
   no. 3, 1 March 1982, US pages 734 - 748 P.C.
   ISAKSON ET AL. 'T cell-derived B cell
   differentiation factor(s).'
 • IMMUNOLOGICAL REVIEWS vol. 67, 1982,
   COPENHAGEN, DK pages 59 - 72 F. SABLITZKY
   ET AL. 'Spontaneous immunoglobulin class
   switching in myeloma and hybridoma cell lines
   differs from physiological class switching'
 • DATABASE WPIL Week 8747, Derwent
   Publications Ltd., London, GB; AN 87-330756
   (47)
 • DATABASE WPIL Week 8913, Derwent
   Publications Ltd., London, GB; AN 89-098035
   (13)
 • DATABASE WPIL Week 9207, Derwent
   Publications Ltd., London, GB; AN 92-053913
   (07)
 • MIYAKE M. ET AL.: 'Generation of two murine
   monoclonal antibodies that can discriminate
   N-Glycolyl and N-Acetyl Neuraminic acid
   residues of GM2 gangliosides' CANCER
   RESEARCH vol. 48, 1998, pages 6154 - 6160
 • MIYAKE M. ET AL.: "The abnormal occurrence
   and the differentiation -dependent distribution
   of N-acetyl and N-glycolyl species of the
   ganglioside GM2 in human germ cell tumors."
   CANCER, Vol. 65, pp. 499-505, 1990

**Description**

FIELD OF THE INVENTION

This invention relates to a method of producing hybridomas which comprises converting (class-switching) a hybridoma capable of producing a monoclonal antibody belonging to the IgM class to a hybridoma capable of producing a monoclonal antibody belonging to the IgG class. The monoclonal antibodies of the IgG class as produced by said hybridomas can be used in the treatment of various diseases more widely as compared with monoclonal antibodies of the IgM class.

BACKGROUND OF THE INVENTION

Antibodies are classifiable into the IgA, IgM, IgG, IgD and IgE classes. Furthermore, mouse antibodies of the IgG class can be classified into four subclasses, $G_1$, $G_{2a}$, $G_{2b}$ and $G_3$ (for human antibodies, $G_1$, $G_2$, $G_3$ and $G_4$). When animals are immunized with an antigen, most of the antibodies obtained belong to the IgM or IgG class. IgG molecules have a dimeric structure with a molecular weight of about 160,000 and are relatively easy to handle. On the other hand, IgM molecules are much greater and have a J chain-bridged complicated pentameric structure with a molecular weight of about 900,000, and therefore they have drawbacks: for instance, they are difficult to purify and are difficult to store because of marked tendency toward aggregation, they are readily deactivated due to partial degradation by proteolytic enzymes (proteases) and therefore Fab production therefrom is difficult, chemical modification thereof, such as coupling thereof with an anticancer agent or a toxin, for instance, often results in loss of binding activity (J. W. Goding: Monoclonal Antibodies - Principles and Practice, Academic Press, 1986). As to which are superior in therapeutic effect against cancer, monoclonal antibodies of the IgG class or monoclonal antibodies of the IgM class, detailed investigations were made by I. D. Bernstein et al. using monoclonal antibodies of the IgG class and monoclonal antibodies of the IgM class, each raised against the lymphocytic Thy-1 antigen [R. H. Kennet, T. J. McKearn and K. B. Bechtol (editors): Monoclonal Antibodies, Plenum Press, 1980, pages 275-291]. According to Bernstein et al., monoclonal antibodies of the IgM class were superior in antitumor effect to monoclonal antibodies of the IgG class comparable to the former in reactivity for Thy-1 antigen-positive lymphocytes in an in vitro complement-dependent antitumor effect test while, in an in vivo antitumor effect test in cancer-bearing mice, monoclonal antibodies of the IgG class exhibited a significant antitumor effect but monoclonal antibodies of the IgM class did not show such effect. Furthermore, a blood half-life investigation following administration of isotope-labeled monoclonal antibodies to mice revealed that monoclonal antibodies of the IgM class have a very short blood half-life as compared with monoclonal antibodies of the IgG class. These experimental results indicate that monoclonal antibodies to be clinically useful in humans should be of the IgG class.

It is known that glycolipids are important cell membrane constituents and readily undergo quantitative and qualitative changes upon canceration of cells [Cancer Res., 45, 2405 (1985)]. Monoclonal antibodies to such glycolipids, if available, would be useful in the treatment and diagnosis of cancer and the effect of such monoclonal antibodies has actually been investigated in cancer patients [H. F. Oettgen (ed.): Gangliosides and Cancer, VCH Publishers, 1989]. However, attempts to produce monoclonal antibodies against non-T cell-dependent antigens, such as glycolipids, readily lead to formation of IgM class monoclonal antibodies in most instances while IgG class monoclonal antibodies are hardly formed [A. H. Bartal and Y. Hirshaut (editors): Methods of Hybridoma Formation, Humana Press, 1987]. $GM_2$, a ganglioside, which is a glycolipid containing a sialic acid residue, occurs only in very small amounts in normal cells but in large amounts in cancer cells, for example in the case of small cell lung carcinoma, melanoma or neuroblastoma, for instance, and therefore monoclonal antibodies to $GM_2$ are thought to be effective in the treatment of such kinds of cancer [Lancet, 48, 6154 (1988)]. However, all the monoclonal antibodies to $GM_2$ that have been so far reported are of the IgM class [Cancer Res., 46, 4116 (1986); Proc. Natl. Acad. Sci. U.S.A., 79, 7629 (1982); Cancer Res., 48, 6154 (1988); J. Biol. Chem., 264, 12122 (1989)].

Even when certain monoclonal antibodies of the IgM class have successfully been formed, attempts to form monoclonal antibodies of the IgG class are still made in many instances from the viewpoint that, for therapeutic purposes, it is important to use monoclonal antibodies of the IgG class, as mentioned above.

There are a number of reports on class switching of hybridomas producing monoclonal antibodies belonging to IgG class subtypes (in the case of mice, $IgG_1$, $IgG_{2a}$, $IgG_{2b}$ and $IgG_3$) but, as for the class switching from IgM to IgG, reports are available only on the following two kinds of antigen:

(1) Phosphorylcholine was used as the antigen and monoclonal antibodies thereto were obtained by cloning hybridomas producing a monoclonal antibody of the IgM class on agarose or 96-well plates and selecting hybridomas resulting from spontaneous class switching and producing a monoclonal antibody of the IgG class [Eur. J. Immunol., 13, 614 (1983); J. Immunol. Methods, 74, 307 (1984); J. Immunol., 140, 2675 (1988)].

(2) Escherichia coli-derived liposaccharide was used as the antigen and monoclonal antibodies thereto were obtained by cloning hybridomas producing a monoclonal antibody of the IgM class on 96-well plates and selecting hybridomas resulting from spontaneous class switching and producing a monoclonal antibody of the IgG class [Eur. J. Immunol., 17, 413 (1987); ibid., 19, 131 (1989)].

However, the methods used in these reported works do not compriee positively class-switching hybridomas producing a monoclonal antibody of the IgM class to hybridomas producing a monoclonal antibody of the IgG class but comprise merely selecting hybridomas resulting from spontaneous class switching. Therefore these methods are applicable only when the antigen is such that monoclonal antibodies of the IgG class are readily formed thereto; said methods cannot be applied when the antigen is a glycolipid, for instance, which, as mentioned above, hardly induces the formation of monoclonal antibodies of the IgG class.

Whitmore et al. investigated as to what treatment could lead to high-frequency class switching of lymphoma B cells producing a monoclonal antibody of the IgM class against phosphorylcholine [Int. Immunol., 3, 95 (1991)]. Thus, Whitmore et al. attempted to cultivate lymphoma B cells in the presence of interleukin-4, interleukin-5, interleukin-6, interferon-$\gamma$, TGF-$\beta$, hydroxyurea, cholera toxin, or sheep erythrocytes (an unpurified antigen). As a result, it was found that when TGF-$\beta$ was used, the class switching from IgM class to IgA class occurred with a high frequency and that the use of interleukin-4 or cholera toxin also resulted in an increased frequency of class switching from IgM class to IgA class. On the other hand, for the class switching from IgM to IgG, treatment with interleukin-4, TGF-$\beta$ or cholera toxin tended to be effective to some extent but failed to cause production of a sufficient quantity of immunoglobulin of the IgG class; there was no statistically significant difference as compared with the untreated group. Thus, even for lymphoma B cells, which are thought to be more readily subject to class switching than hybridomas, no method capable of causing high-frequency class switching from IgM to IgG has been reported as yet.

Hybridomas are said to change themselves spontaneously into hybridomas producing a class-switched monoclonal antibody, with a probability of one per tens of thousands to tens of billions [Eur. J. Immunol., 17, 413 (1987)]. For preparing class-switched monoclonal antibodies, it is thus necessary to select a hybridoma that has spontaneously changed in the above manner from among a great mass of unchanged hybridomas and proliferate it alone. For such selection, there is known the sib selection method which comprises cloning hybridomas using an agarose or 96-well plates and searching, one by one, for hybridomas that have changed [J. Immunol., 131, 877 (1983)] and the method comprising utilizing flow cytometry for collecting, at a stroke, several hundred to several thousand hybridoma cells that have changed [J. Immunol. Methods, 52, 1 (1982)]. However, the probability of spontaneous change of hybridomas is itself low, so that either of the methods can hardly be used in the class switching from IgM class to IgG class which is thought to be particularly low in probability. Therefore these methods are not applicable when the antigen is a glycolipid or the like with which monoclonal antibodies of the IgG class are difficult to obtain.

WO-A-90/02795 discloses a method for producing a hybridoma producing IgG mAb characterized by immunizing lymphocytes with T cell-dependent or T cell-independent antigen.

Miyake et al. disclose in Cancer Research 1988, Vol. 48, pages 6154 - 6160 and Cancer 1990, Vol. 65, pages 499 - 505 production of monoclonal antibodies of the type IgM which detect N-acetyl or N-glycolyl $GM_2$ gangliosides.

SUMMARY OF THE INVENTION

Accordingly, it is an object of the invention to provide a method of producing hybridomas capable of producing a monoclonal antibody of the IgG class against a glycolipid or the like antigen.

The present invention provides a method of producing hybridomas capable of producing a monoclonal antibody of the IgG class which comprises cultivating a hybridoma producing a monoclonal antibody of the class IgM in the presence of an antigen reactive with said antibody and in the presence of thymocytes; and isolating the IgG producing hybridomas. The invention also provides hybridomas capable of producing a monoclonal antibody belonging to the IgG class and being reactive with N-acetyl-$GM_2$, and antibodies produced by said hybridomas; as well as pharmaceutical compositions containing said antibodies; and the use of said antibodies for preparing pharmaceutical compositions for treating tumors.

BRIEF DESCRIPTION OF THE DRAWINGS

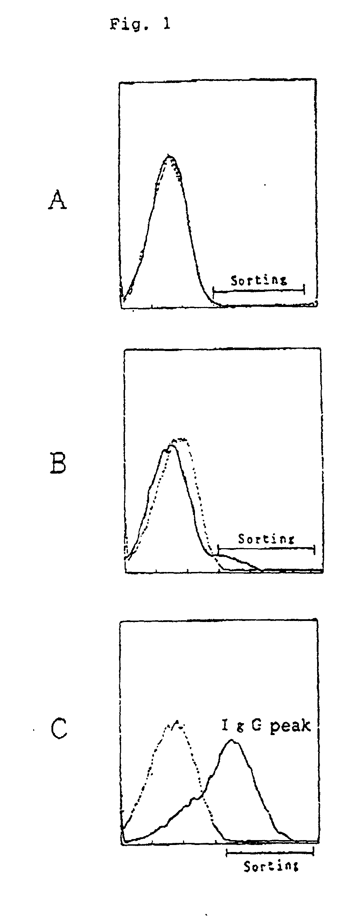

Fig. 1 shows the results of fluorescence intensity measurement by flow cytometry following reaction of FITC-antiprotein A with the hybridoma KM-697. The ordinate is for the number of cells and the abscissa for the fluorescence intensity. The solid line shows the pattern for the hybridoma reacted with FITC-anti-protein A while the dotted line shows the pattern for the hybridoma not subjected to reaction with FITC-anti-protein A. A shows the pattern for the hybridoma KM-697 free from antigen stimulation, B shows the pattern for the hybridoma KM-697 after five repetitions of antigen stimulation, and C shows the pattern for the hybridoma KM-697 after six repetitions of antigen stimulation.

Fig. 2 shows the pattern of SDS polyacrylamide gel electrophoresis of a purified sample of the monoclonal antibody KM-750 (using a 4-15 gradient gel). From the left are shown the electrophoretic patterns of a molecular weight marker, a standard sample of IgG, a standard sample of IgM, and the monoclonal antibody KM-750, respectively. A shows the results of electrophoresis performed under reducing conditions while B shows the results of electrophoresis performed under non-reducing conditions.

Fig. 3 shows the reactivities of some serially diluted (0.05-25 µg/ml) purified monoclonal antibodies for 20 pmol/well of $GM_2$. The symbol □ is used for KM-750, o for KM-796, and ∆ for KM-737.

Fig. 4 shows the reactivities of some purified monoclonal antibodies at a concentration of 10 µg/ml for serial dilutions (0.04-20 pmol/well) of $GM_2$. The symbol □ is used for KM-750, o for KM-796, and ∆ for KM-737.

Fig. 5 shows complement dependent cytotoxicity of the monocloanl antibodies against neurocytoma IMR32 which expresses ganglioside $GM_2$. The abscissa stands for the concentration of the antibody added. The symbol • is used for KM-750 and o for KM-737.

DETAILED DESCRIPTION OF THE INVENTION

The subject invention relates to hybridomas producing monoclonal antibodies of the IgG class which are produced by cultivating a hybridoma producing a monoclonal antibody belonging to the IgM class in the presence of an antigen reactive with the IgM antibody, and in the presence of thymocytes.

(1) Stimulation of hybridomas with an antigen

$1 \times 10^4$ to $1 \times 10^6$ cells per milliliter of a hybridoma strain producing a monoclonal antibody belonging to the IgM class are cultivated in the presence of thymocytes. As the hybridoma producing a monoclonal antibody belonging to the IgM class, there may be mentioned, for example, hybridomas producing a monoclonal antibody against a glycolipid such as the ganglioside $GM_2$, sialylated $Le^x$, $Le^x$ or $Le^Y$. As the hybridoma producing a monoclonal antibody against a glycolipid which is used as the antigen, there may be mentioned, among others, the rat hybridomas KM-602, KM-603, KM-604, KM-605 and KM-606 and the mouse hybridomas KM-531, KM-693, KM-694, KM-695, KM-696 and KM-697, each capable of producing a monoclonal antibody reactive with the ganglioside $GM_2$. The hybridoma belonging to the IgM class used in the present invention can be prepared by production methods as conventionally used in preparation of known hybridoma strains. For example, the rat hybridomas KM-602, KM-603, KM-604, KM-605 and KM-606 and mouse hybridomas KM-531, KM-693, KM-694, KM-695, KM-696 and KM-697 can be prepared by the same method as described in JP-A-4-1138 or a modified one. The hybridomas KM-531 and KM-603 have been deposited since September 14, 1989 and October 31, 1989, respectively, and KM-696 and KM-697 since April 2, 1991, with the Fermentation Research Institute, Agency of Industrial Science and Technology of 1-3, Higashi 1-chome, Tsukuba-shi, Ibaraki, Japan under the deposit numbers FERM BP-2597, FERM BP-2636, PERM BP-3337 and FERM BP-3338, respectively in accordance with the Budapest Treaty. As the thymocytes, there may be mentioned mouse-derived thymocytes which mainly comprises T cells, macrophages and dendritic cells, and these are used at $1 \times 10^6$ to $1 \times 10^8$ cells/ml. When a hybridoma is cultivated in the presence of thymocytes, antigen stimulation is effected by adding an antigen reactive with the antibody produced by said hybridoma to the culture fluid. The antigen concentration is 10 to $1 \times 10^3$ ng/ml, and the cultivation period required for one antigen stimulation is 5 to 7 days. The antigen is added to the culture fluid preferably in a form encapsulated in liposomes. The liposomes are prepared by using a phospholipid, such as dipalmitoylphosphatidylcholine or dipalmitoylphosphatidylic acid, and cholesterol. It is preferable to use a combination of several kinds of phospholipid and cholesterol, The addition of a mitogen, such as lipid A, to the liposomes may lead to more effective antigen stimulation. Generally, only one antigen stimulation is insufficient but about 6 to 10 repetitions of antigen stimulation are required.

(2) Isolation of hybridomas resulting from class switching

From among the hybridomas subjected to antigen stimulation as described under (1), hybridomas producing a monoclonal antibody of the IgG class are isolated. For the isolation, the sib selection method comprising cloning hybridomas using an agarose medium or 96-well plates and checking one by one in search of hybridomas that have changed [J. Immunol., 131, 877 (1983)] and the method comprising using flow cytometry for collecting several hundred to several thousand hybridoma cells that have changed [J. Immunol. Methods, 52, 1 (1982)] may be used. For efficient isolation, the method involving flow cytometry is preferred, hence said method is described below in further detail.

For isolation by flow cytometry, it is necessary that the surface of hybridoma cells to be subjected to class switching should carry immunoglobulin molecules belonging to the same class and having the same binding specificity as the monoclonal antibody secreted by said cells. Therefore, prior to stimulation with an antigen, whether immunoglobulin molecules are present on the cell surface or not is examined by using an anti-IgM antibody labeled with a fluorescent dye such as FITC (fluorescein isothiocyanate) (hereinafter, FITC-anti-IgM). Thus, FITC-anti-IgM is reacted with hybridomas and the cell surface fluorescence intensity is measured by observation under a fluorescence microscope or by flow cytometric analysis, and hybridomas carrying a sufficient number of immunoglobulin molecules of the IgM class on the cell surface are selected.

After completion of the antigen stimulation described under (1), a fluorescent dye-labeled anti-IgG antibody (hereinafter, FITC-anti-IgG), fluorescent dye-labeled protein A (hereinafter, FITC-protein A) or fluorescent dye-labeled protein G (hereinafter, FITC-protein G) is reacted with ten million to thousand million hybridomas. After reaction, the hybridomas are subjected to flow cytometry and fluorescence intensity measurement is performed following exposure to laser beams. Positive or negative electric charge is given to hybridomas showing a high fluorescence intensity and letting the hybridomas flow between deflector plates at a high velocity to selectively collect charged hybridomas. In case the number of repetitions of antigen stimulation is insufficient, there are detectable few hybridomas showing a high fluorescence intensity. At any rate, a group of cells (several percent of all the hybridoma cells) that show shifting to the higher fluorescence intensity side relative to a distribution curve prepared by plotting the fluorescence intensity (on the abscissa) against the number of cells (on the ordinate) are collected and again subjected to antigen stimulation, followed by flow cytometry. As the number of repetitions of antigen stimulation increases, a cell group showing a high fluorescence intensity appears more distinctly and the distribution curve shows two peaks. At this stage, cells belong to the peak of the higher fluorescence intensity are collected.

(3) Cloning

The hybridoma cells obtained as described under (2) and showing a high fluorescence intensity are diluted to an appropriate cell density, distributed into wells of 96-well plates and cultivated. The cell density should preferably be one cell per one well or per two wells (cloning by the limiting dilution method). For attaining a better cultivation efficiency, the cultivation should preferably be carried out in the presence of $1 \times 10^4$ to $1 \times 10^7$ thymocytes per milliliter. After 1 to 2 weeks of cultivation, the culture supernatants are sampled and the proportion of wells in which a monoclonal antibody of the IgG class is produced and the proportion of wells in which a monoclonal antibody of the IgM class is produced are examined by enzyme immunoassay, for instance. In case wells in which a monoclonal antibody of the IgM class is produced are found, the hybridomas in the wells in which a monoclonal antibody of the IgG class is produced are again cloned by the limiting dilution method. The cloning by the limiting dilution method is repeated in that manner until wells in which a monoclonal antibody of the IgM class are no longer found and the production of a monoclonal antibody of the IgG class is detected in not less than 90% of the wells. Finally, the hybridomas in those wells in which a monoclonal antibody of the IgG class is produced most abundantly are selected as class-switched hybridomas. As the class-switched hybridomas capable of producing a monoclonal antibody of the IgG class, there may be mentioned hybridomas producing a monoclonal antibody against the ganglioside $GM_2$, such as KM-750 and KM-796. The hybridomas KM-750 and KM-796 have been deposited, since April 2, 1991, with the Fermentation Research Institute, Agency of Industrial Science and Technology under the deposit numbers FERM BP-3339 and FERM BP-3340, respectively in accordance with the Budapest Treaty.

(4) Preparation of monoclonal antibodies

The class-switched hybridomas selected as described under (3) are cultured for 2 to 7 days using roller bottles or spinners. Each culture supernatant is collected by centrifugation and applied to a protein A column or a protein G column. The IgG class monoclonal antibody adsorbed is then eluted. As the thus-purified monoclonal antibody, there may be mentioned, for example, the monoclonal antibodies KM-750 and KM-796 produced against the ganglioside $GM_2$ by the hybridomas KM-750 and KM-796, respectively. That the monoclonal antibody obtained belongs to the IgG class is checked by SDS electrophoresis, for instance. It is also possible to prepare such a purified antibody by intraperitoneally administering 0.5 ml of pristane (2,6,10,14-tetramethylpentadecane) to mice, feeding them for 3 days to 2 weeks, then intraperitoneally administering the hybridoma to them, collecting the resulting ascitic fluid therefrom, and purifying the antibody contained in the ascitic fluid using a protein A or protein G column. The subclass of the monoclonal antibody can be determined by enzyme immunoassay using a subclass typing kit or by the Ouchterlony method which is based on immune precipitation. The quantity of protein is determined by the Lowry method or by calculation based on the optical density at 280 nm.

(5) Investigation of the activity and reaction specificity of the monoclonal antibodies

The activity and reaction specificity of each monoclonal antibody obtained are examined by enzyme immunoassay or radioimmunoassay (E. Harlow and D. Lane (editors): Antibodies - A Laboratory Manual, Cold Spring Harbor Laboratory, 1988).

The monoclonal antibodies according to the present invention can be used alone as an antitumor agent. They may be formulated into an antitumor composition together with at least one pharmaceutically acceptable carrier. For instance, the monoclonal antibodies are dissolved in physiological saline, an aqueous solution of glucose, lactose or mannitol and the like. The powder of the monoclonal antibodies for injection can be prepared by lyophilizing the monoclonal antibodies in accordance with the conventional method and mixing the lyophilized products with sodium chloride. The antitumor composition may further contain additives conventionally used well known in the art of medical preparation, for example, pharmaceutically acceptable salts.

The monoclonal antibodies according to the present invention can be administered in the form of the above-described antitumor composition to mammals including human in a dose of 0.2 to 20 mg/kg/day. The dose may vary depending on the age, condition, etc. of patients. The administration of the antitumor composition can be effected by intravenous injection once a day (single administration or consecutive administration) or intermittently one to three times a week or once every two to three weeks.

The antitumor composition is expected to be useful for treating tumors such as lung carcinoma, cerebroma and melanoma.

The following examples are further illustrative of the present invention.

EXAMPLE

(1) Hybridoma selection

The rat hybridomas KM-602, KM-603, KM-604, KM-605 and KM-606 and the mouse hybridomas KM-531, KM-693, KM-694, KM-695, KM-696 and KM-697, each producing a monoclonal antibody of the IgM class which is reactive with the ganglioside $GM_2$ were prepared according to the method described in JP-A-4-1138 and were examined for the presence or absence of an immunoglobulin on the cell surface. Thus, for each hybridoma, 200 μl of a 32-fold dilution of FITC-anti-mouse IgG (H+L) (0.5 mg/ml; Funakoshi) or a 200-fold dilution of FITC-anti-rat IgG (H+L) (DAKO) was added to about $10^6$ cells and the reaction was carried out at 4°C for 30 minutes. Then, the hybridoma cells were washed with two portions of cooled PBS (phosphate buffered saline, pH 7.2) and suspended in about 6 ml of PBS, and the fluorescence intensity was measured using a flow cytometer (FCS-1, Nippon Bunko). As a result, neither immunoglobulins of the IgM class nor immunoglobulins of the IgG class were found on the cell surface of any of the hybridomas KM-602, KM-603, KM-604, KM-605 and KM-606. As for the hybridomas KM-531, KM-693, KM-694, KM-695, KM-696 and KM-697, IgM class immunoglobulin signals were observed on the cell surface in a sufficient number for cell collection by flow cytometry. However, in these hybridomas, the cell surface was quite free of any immunoglobulin of the IgG class reactive with FITC-anti-mouse γ chain (Funakoshi). Among the cell surface immunoglobulin-positive hybridomas, KM-696 and KM-697, which showed high reactivity with the antigen and high antigen specificity, were selected for the subsequent experiments. The monoclonal antibody KM-696 produced by the hybridoma KM-696 specifically reacted with N-acetyl-$GM_2$ but did not react with other gangliosides such as N-acetyl-$GM_3$, N-glycolyl-$GM_3$, N-glycolyl-$GM_2$, $GM_1$, $GD_1$, $GD_2$, $GD_3$, $GD_{1a}$, $GD_{1b}$, $GT_{1b}$ and $GQ_{1b}$. The monoclonal antibody KM-697 produced by the hybridoma KM-697 specifically reacted with N-acetyl-$GM_2$ and N-glycolyl-$GM_2$ but did not react with other gangliosides such as N-acetyl-$GM_3$, N-glycolyl-$GM_3$, $GM_1$, $GD_1$, $GD_2$, $GD_3$, $GD_{1a}$, $GD_{1b}$, $GT_{1b}$ and $GQ_{1b}$.

(2) Antigen stimulation of hybridomas and isolation by flow cytometry

First, $GM_2$-containing liposomes were prepared in the following manner. In chloroform were dissolved 0.5 μmol of dipalmitoylphosphatidylcholine (Sigma), 0.05 μmol dipalmitoylphosphatidylic acid (Sigma), 0.5 μmol of cholesterol (Nakarai Tesque), 2.5 μg of lipid A (Funakoshi) and 2.0 μg of the ganglioside $GM_2$ (Boehringer Mannheim). The solvent was then distilled off under reduced pressure to give a thin film. Thereto was added 0.5 ml of PBS. The vessel contents were heated at 50 to 60°C and then subjected to stirring with a mixer and 10 minutes of ultrasonic treatment to give $GM_2$-containing liposomes.

Then, 20 to 30 million cells of the hybridoma KM-696 or KM-697 not yet stimulated with the antigen were allowed to react with 200 μl of a 20-fold dilution of FITC-anti-protein A (5 mg/ml; Boehringer Mannheim) at 4°C for 30 minutes. The hybridoma cells were washed twice with cooled PBS, suspended in 6 ml of PBS and subjected to flow cytometry.

In flow cytometry, a group of several hundred thousand cells showing shifting to the higher fluorescence intensity side on a distribution curve prepared by plotting the fluorescence intensity (abscissa) against the number of cells (ordinate) were collected although any group of hybridoma cells showing a high fluorescene intensity could not be detected.

Using a 24-well plate (Nunc), the several hundred thousand cells of each hybridoma as collected in the above manner were cultured in 1 to 1.5 ml of RPMI 1640 medium (Nissui Pharmaceutical) supplemented with 10% fetal calf serum (CSL) in the presence of mouse thymocytes (the number of cells being about one fiftieth of the total number of thymocytes of a 3- to 4-week old mouse). On that occasion, antigen stimulation was effected by adding the $GM_2$-containing liposomes to a final $GM_2$ concentration of 500 ng/ml. In control runs, cultivation was performed without antigen stimulation, then the cells were reacted with FITC-anti-protein A and subjected to flow cytometry, and this procedure was repeated 15 times for each hybridoma. In this cultivation without antigen stimulation, no group of cells reacting with FITC-anti-protein A and giving an increased fluorescence intensity appeared.

The cultivation for antigen stimulation in the presence of $GM_2$-containing liposomes was conducted at 37°C for 6 to 7 days in the presence of 5% $CO_2$. After antigen stimulation and when the number of hybridoma cells reached twenty to thirty million, the cells are reacted with FITC-anti-protein A, then washed with PBS, suspended in 6 ml of PBS and subjected to flow cytometry to thereby collect a group of several hundred thousand cells showing shifting to the higher fluorescence intensity side. After three repetitions of the cultivation for antigen stimulation followed by sorting by flow cytometry, the cultivation without antigen stimulation followed by sorting by flow cytometry was repeated two times for the hybridoma KM-696 and three times for the hybridoma KM-697. For each hybridoma, the cultivation with antigen stimulation followed by sorting by flow cytometry was repeated further two times, whereupon a group of cells reacting with FITC-anti-protein A and giving an increased fluorescence intensity appeared for both the hybridomas KM-696 and KM-697. Both the cell groups were sorted out by flow cytometry and subjected once more to antigen stimulation and cultivation, after which all cells of the hybridoma KM-696 and all cells of the hybridoma KM-697 belonged to the respective cell groups reacting with FITC-anti-protein A and giving a high fluorescence intensity. Fig. 1 shows the fluorescence intensity pattern in flow cytometry of the hybridoma KM-697.

(3) Cloning

With both the hybridomas KM-696 and KM-697, continued cultivation of the group of cells reacting with FITC-anti-protein A and giving a high fluorescence intensity resulted in reappearance of a group of cells not reactive with FITC-anti-protein A and then in an increase in the number of such cells. Therefore, each group of cells reacting with FITC-anti-protein A and giving a high fluorescence intensity was subjected to cloning. First, cells were distributed into wells of 96-well plates in the form of a diluted cell suspension such that each well or each two wells contained one cell and cultured in the presence of about one million thymocytes. After about 10 days of cultivation, the culture supernatants were sampled. The samples were tested by the enzyme immunoassay technique mentioned below and the proportion of wells in which a monoclonal antibody of the IgG class was produced and that of wells in which a monoclonal antibody of the IgM class was produced were examined. In the first cloning, wells in which a monoclonal antibody of the IgM class were found. Therefore, the hybridomas in wells in which a monoclonal antibody of the IgG class was produced were again subjected to cloning by the limiting dilution method. In the second cloning, no monoclonal antibody of the IgM class was produced in any of the wells and the production of a monoclonal antibody of the IgG class was noted in not less than 90% of the wells. Finally, the wells in which a monoclonal antibody of the IgG class was produced most abundantly were selected. Thus the hybridoma KM-750 was selected as a product of class switching from the hybridoma KM-697, and the hybridoma KM-796 as a product of class switching from the hybridoma KM-696.

Enzyme immunoassay (ELISA)

$GM_2$ (2 ng; Boehringer Mannheim), or 2 ng of any of other gangliosides, was dissolved in 2 μl of an ethanol solution containing 5 ng of phosphatidylcholine (Sigma) and 2.5 ng of cholesterol (Sigma). This solution or a dilution thereof was distributed in 20-μl portions into wells of a 96-well microtiter plate (Greiner) and, after air drying, blocking was effected using PBS containing 1% BSA. A hybridoma culture supernatant or a purified monoclonal antibody was added to the plate (50 to 100 μl per well) and the reaction was carried out at 4°C for 10 hours. Then, the wells were washed well with PBS, then peroxidase-labeled anti-mouse gamma chain antibody (Funakoshi) or peroxidase-labeled protein A (Funakoshi) was added (50 to 100 μl per well), and the reaction was carried out at room temperature for 1 to 2 hours. After washing with PBS, 50 to 100 μl of an ABTS substrate solution [prepared by dissolving 550 mg of 2,2'-azinobis(3-ethylbenzothiazoline-6-sulfonic acid) diammonium salt in 0.1 M citrate buffer (pH 4.2) and adding, just prior to use, hydrogen peroxide in an amount of 1 μl/ml] was added to each well for color development. The optical density at 415 nm ($OD_{415}$) was then measured.

(4) Preparation of monoclonal antibodies

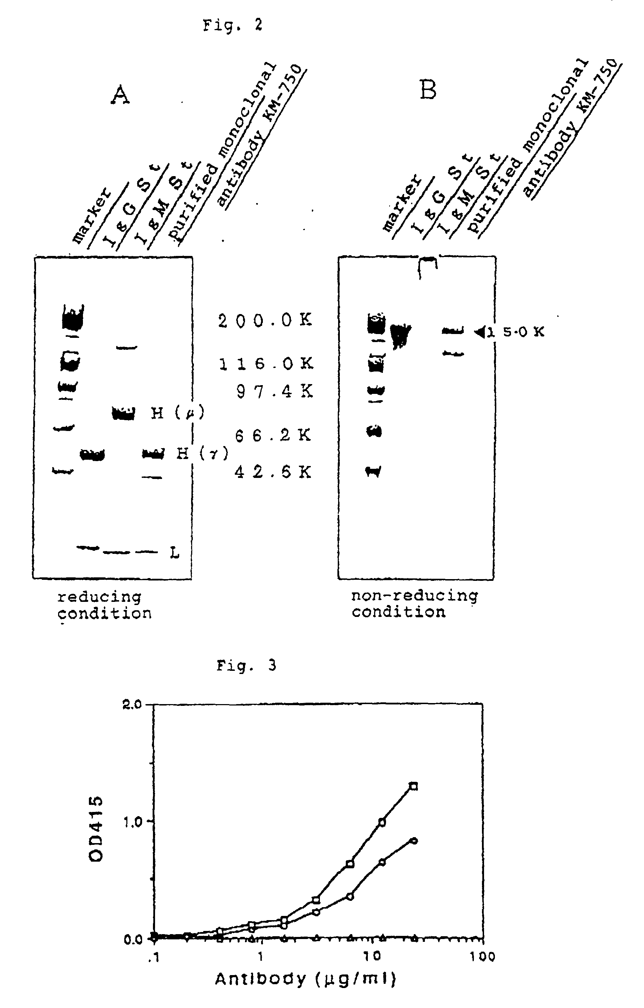

The hybridomas KM-750 and KM-796 were respectively cultured in 1 to 2-liter spinner incubators for 3 days to give culture supernatants in large amounts. Each culture supernatant collected was applied to a protein A-Sepharose 4B column (Pharmacia) and the IgG class monoclonal antibody adsorbed was eluted with 0.1 M citrate buffer (pH 4.0). The eluate was dialyzed against PBS to give a purified monoclonal antibody. SDS polyacrylamide gel electrophoresis proved that the purified monoclonal antibody was of the IgG class. The gel used was a 4-15% gradient gel (Daiichi Kagaku). Fig. 2 Shows the results of SDS polyacrylamide gel electrophoresis of KM-750. Antibody subclass determination by enzyme immunoassay using a mouse subclass typing kit (Zymed) revealed that the monoclonal antibody KM-750 produced by the hybridoma KM-750 and the monoclonal antibody KM-796 produced by the hybridoma KM-796 were both of the $IgG_3$ subclass. Protein quantitation was carried out using a BCA reagent (Pierce).

(5) Activity assay of monoclonal antibodies

The purified monoclonal antibodies were each tested, in serial dilutions (0.05 to 25 µg/ml), for reactivity with 20 pmol/well of $GM_2$ by the ELISA technique. For comparison, the reactivity with $GM_2$ of the monoclonal antibody KM-737 (of the $IgG_3$ class) which is a monoclonal antibody against salmon growth hormone, was also evaluated. As shown in Fig. 3, the monoclonal antibodies KM-750 and KM-796 both showed a high level of binding to $GM_2$ in an antibody concentration-dependent manner, whereas the monoclonal antibody KM-737 was not bound to $GM_2$.

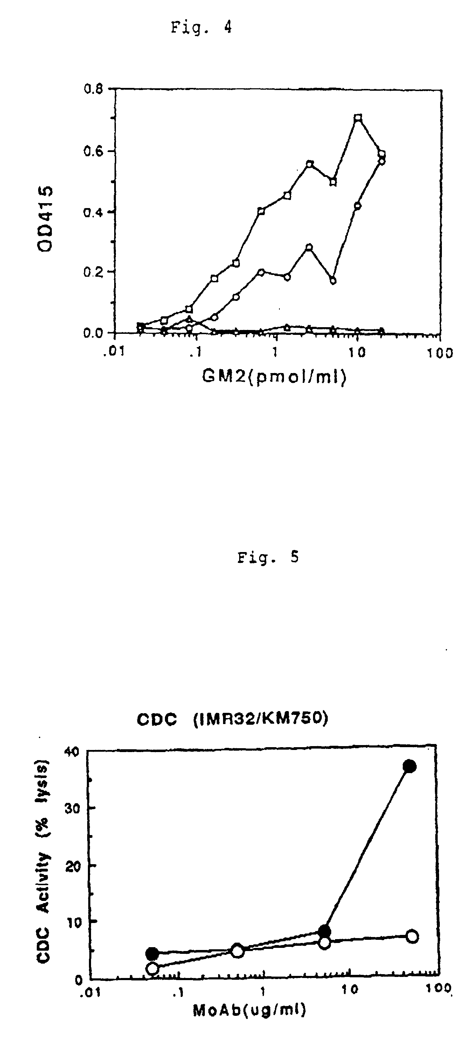

Then, the reactivity of each purified monoclonal antibody at a concentration of 10 µg/ml with serial dilutions (0.04 to 20 pmol/well) of $GM_2$ was estimated by the ELISA method. For comparison, the reactivity of the monoclonal antibody KM-737 was also investigated. As shown in Fig. 4, both the monoclonal antibodies KM-750 and KM-796 showed a high $GM_2$ binding activity in an antigen concentration-dependent manner while the monoclonal antibody KM-737 showed no $GM_2$ binding activity.

(6) Testing for reaction specificity

The monoclonal antibodies KM-750 and KM-796 were tested for reactivity with the gangliosides $GM_1$, N-acetyl-$GM_2$ (Boehringer Mannheim), N-glycolyl-$GM_2$, N-acetyl-$GM_3$, N-glycolyl-$GM_3$, $GD_{1a}$, $GD_{1b}$ (Iatron), $GD_2$, $GD_3$, (Iatron), $GT_{1b}$ (Funakoshi) and $GQ_{1b}$ (Iatron) by the ELISA method. $GM_1$ and $GD_{1a}$ were purified from the bovine brain, N-glycolyl-$GM_2$ and N-glycolyl-$GM_3$ from the mouse liver, and N-acetyl-$GM_3$ was purified from canine erythrocytes and $GD_2$ from the cultured cell line IMR32 (ATCC CCL127), essentially by a per se known method [J. Biol. Chem., 263, 10915 (1988)]. The results are shown in Table 1. As shown in Table 1, KM-750 reacted only with N-acetyl-$GM_2$ and N-glycolyl-$GM_2$ but did not react with the other gangliosides. This reaction specificity is the same as that of the monoclonal antibody KM-697 before class switching, indicating that the reaction specificity remained unchanged even after class switching to IgG. KM-796 reacted only with N-acetyl-$GM_2$ but did not react with the other gangliosides. This reaction specificity is the same as that of the monoclonal antibody KM-696 before class switching and thus, for KM-796 as well, the reaction specificity remained unchanged even after class switching to IgG.

Table 1

| Ganglioside | $OD_{415}$ | |
| --- | --- | --- |
| | KM-750 | KM-796 |
| N-Acetyl-$GM_3$ | 0.099 | 0.031 |
| N-Glycolyl-$GM_3$ | 0.052 | 0.014 |
| N-Acetyl-$GM_2$ | 1.239 | 0.579 |
| N-Glycolyl-$GM_2$ | 0.594 | 0.022 |
| $GM_1$ | 0.039 | 0.004 |
| $GD_2$ | 0.077 | 0.035 |
| $GD_3$ | 0.044 | 0.010 |
| $GD_{1a}$ | 0.056 | 0.019 |
| $GD_{1b}$ | 0.038 | 0.004 |
| $GT_{1b}$ | 0.067 | 0.044 |
| $GQ_{1b}$ | 0.021 | 0.017 |

(7) Testing for complement dependent cytotoxicity (CDC)

(a) Preparation of target cells

Neurocytoma IMR32 (ATCC CCL 127) which expresses the ganglyoside $GM_2$ was suspended in the RPMI1640 medium supplemented with 10% fetal calf serum (FCS) and adjusted to a cell density of $1 \times 10^7$ cells/ml to serve as target cells. $Na_2{}^{51}CrO_4$ was added thereto to 100 µCi/l $\times 10^7$ cells and the medium was incubated at 37°C for 1 hour. The cells were washed with the RPMI1640 medium supplemented with 10% FCS three times. The whole cells were allowed to stand at 4°C for 30 minutes in the RPMI1640 medium supplemented with 10% FCS for dissociation. After centrifugation at 1,200 rpm for 10 minutes, the cell density was adjusted to $4 \times 10^6$ cells/ml by adding the RPMI1640 medium supplemented with 10% FCS.

(b) Preparation of complement

The target cells prepared in above (a) ($2 \times 10^7$ cells) were added to 1 ml of rabbit complement (Cederane Laboratory) and allowed to react at 4°C for 1 hour. The cells were filtered off to give a complement solution.

(c) Measurement of CDC activity

The monoclonal antibodies KM-750 and KM-737 were each added to the wells of the 96-well U-bottom plate to a final concentration ranging within 0.05 to 50 µg/ml and further $2 \times 10^5$ cells of the target cells were added to each well. After a reaction at room temperature for 1 hour, centrifugation was carried out at 1,200 rpm for 5 minutes to remove the supernatant. The complement solution prepared in above (b) was 9-fold diluted with the RPMI1640 medium supplemented with 10% FCS and a 150 µl portion of the dilution was added to each well. A reaction was carried out at room temperature for 1 hour and then the cells were removed by centrifugation at 1,200 rpm for 5 minutes. The amount of $^{51}$Cr in 75 µl of the supernatant was determined by a γ-counter. The amount of naturally dissociated $^{51}$Cr was determined in the same manner as described above except that the RPMI1640 medium supplemented with 10% FCS alone, containing neither monoclonal antibody nor complement, was added to the target cells. The total amount of dissociated $^{51}$Cr was determined in the same manner as described above except that 5N sodium hydroxide solution in place of the monoclonal antibody and the complement solution was added to the target cells. CDC activity was calculated by the following equation.

$$\text{CDC activity (\%)} = \frac{\text{Amount of } ^{51}\text{Cr in supernatant of specimen - Amount of naturally dissociated } ^{51}\text{Cr}}{\text{Total amount of dissociated } ^{51}\text{Cr - Amount of naturally dissociated } ^{51}\text{Cr}} \times 100$$

The results are shown in Fig. 5. As shown in Fig. 5, it can be seen that the monoclonal antibody KM-750 has CDC activity against neurocytoma IMR32.

As detailedly described hereinabove, the present invention provides a method of producing hybridomas which comprises subjecting hybridomas producing a monoclonal antibody of the IgM class to class switching to hybridomas producing a monoclonal antibody of the IgG class. The monoclonal antibodies of the IgG class produced by said hybridomas can be used widely in the treatment of various diseases.

**Claims**

1. A method of producing a hybridoma capable of producing a monoclonal antibody belonging to the class IgG which comprises

   (1) cultivating a hybridoma capable of producing a monoclonal antibody belonging to the IgM class, in the presence of an antigen reactive with the latter antibody and in the presence of thymocytes; and

   (2) isolating the IgG producing hybridomas.

2. The method of claim 1, wherein the isolated hybridomas are further cultivated, optionally in the presence of thymocytes.

3. The method of claim 1, wherein the isolated hybridomas are cloned by limiting dilution method.

4. The method of anyone of Claims 1 to 3, wherein the hybridoma capable of producing a monoclonal antibody belonging to the IgM class is a hybridoma capable of producing a monoclonal antibody reactive with N-acetyl-GM$_2$ and/or N-glycolyl-GM$_2$.

5. The method of anyone of Claims 1 to 3, wherein the hybridoma capable of producing a monoclonal antibody belonging to the IgM class is selected from the group consisting of the hybridomas KM-531 (FERM BP-2597), KM-696 (FERM BP-3337) and KM-697 (FERM BP-3338).

6. The method of anyone of claims 1 to 5, wherein the antigen is added to the hybridoma culture in the form of liposomes.

7. A hybridoma capable of producing a imonoclonal antibody which belongs to the IgG class and is reactive with N-acetyl-GM$_2$.

8. A hybridoma capable of producing a monoclonal antibody which belongs to the IgG class and is reactive with N-acetyl-GM$_2$ and N-glycolyl-GM$_2$.

9. The hybridoma cell line KM-796 (FERM BP-3340).

10. The hybridoma cell line KM-750 (FERM BP-3339).

11. A monoclonal antibody belonging to the IgG class and reactive with N-acetyl-GM$_2$.

12. The monoclonal antibody of claim 11 which is produced by the hybridoma cell line KM-796 (FERM BP-3340).

13. A monoclonal antibody belonging to the IgG class and reactive with N-acetyl-GM$_2$ and N-glycolyl-GM$_2$.

14. The monoclonal antibody of claim 13 which is produced by the hybridoma cell line KM-750 (FERM BP-3339).

15. A pharmaceutical composition containing a monoclonal antibody belonging to the IgG class being reactive with N-acetyl-GM$_2$ and a pharmaceutically acceptable carrier.

16. A pharmaceutical composition containing a monoclonal antibody belonging to the IgG class being reactive with N-acetyl-GM$_2$ and N-glycolyl-GM$_2$ and a pharmaceutically acceptable carrier.

17. Use of monoclonal antibody of claim 11, for the manufacture of a pharmaceutical composition for treating tumors.

18. Use of a monoclonal antibody of claim 13 for the manufacture of a pharmaceutical composition for treating tumors.

**Patentansprüche**

1. Verfahren zur Herstellung eines Hybridoms, welches zur Produktion eines monoklonalen Antikörpers der Klasse IgG befähigt ist, umfassend:

   (1) die Kultivierung eines Hybridoms, welches zur Produktion eines monoklonalen Antikörpers der Klasse IgM befähigt ist, in Gegenwart eines Antigens, das mit letzterem Antikörper reagiert sowie in Gegenwart von Thymocyten; und
   (2) Isolierung der IgG-produzierenden Hybridome.

2. Verfahren nach Anspruch 1, worin die isolierten Hybridome außerdem, gegebenenfalls in Gegenwart von Thymocyten, kultiviert werden.

3. Verfahren nach Anspruch 1, worin die isolierten Hybridome unter Anwendung der limitierenden Verdünnungsmethode kloniert werden.

4. Verfahren nach einem der Ansprüche 1 bis 3, worin das Hybridom, das die Fähigkeit zur Produktion eines monoklonalen Antikörpers der Klasse IgM besitzt, ein Hybridom ist, welches die Fähigkeit besitzt, einen monoklonalen Antikörper zu produzieren, der mit N-Acetyl-GM$_2$ und/oder N-Glycolyl-GM$_2$ reagiert.

5. Verfahren nach einem der Ansprüche 1 bis 3, worin das Hybridom, welches die Fähigkeit zur Produktion eines Antikörpers der Klasse IgM besitzt, ausgewählt ist unter den Hybridomen KM-531 (FERM BP-2597), KM-696 (FERM BP-3337) und KM-697 (FERM BP-3338).

6. Verfahren nach einem der Ansprüche 1 bis 5, worin das Antigen der Hybridom-Kultur in Form von Liposomen zugesetzt wird.

**7.** Hybridom, welches die Fähigkeit zur Produktion eines monoklonalen Antikörpers der Klasse IgG besitzt und mit N-Acetyl-GM$_2$ reagiert.

**8.** Hybridom, welches die Fähigkeit zur Produktion eines monoklonalen Antikörpers der Klasse IgG besitzt, welcher mit N-Acetyl-GM$_2$ und N-Glycolyl-GM$_2$ reagiert.

**9.** Die Hybridomzelllinie KM-796 (FERM BP-3340).

**10.** Die Hybridomzelllinie KM-750 (FERM BP-3339).

**11.** Monoklonaler Antikörper der Klasse IgG, der mit N-Acetyl-GM$_2$ reagiert.

**12.** Monoklonaler Antikörper nach Anspruch 11, der von der Hybridomzelllinie KM-796 (FERM BP-3340) produziert wird.

**13.** Monoklonaler Antikörper der Klasse IgG, der mit N-Acetyl-GM$_2$ und N-Glycolyl-GM$_2$ reagiert.

**14.** Monoklonaler Antikörper nach Anspruch 13, der von der Hybridomzelllinie KM-750 (FERM BP-3339) produziert wird.

**15.** Pharmazeutisches Mittel, enthaltend einen monoklonalen Antikörper der Klasse IgG, der mit N-Acetyl-GM$_2$ reagiert, sowie einen pharmazeutisch verträglichen Träger.

**16.** Pharmazeutisches Mittel, enthaltend einen monoklonalen Antikörper der Klasse IgG, der mit N-Acetyl-GM$_2$ und N-Glycolyl-GM$_2$ reagiert, und einen pharmazeutisch verträglichen Träger.

**17.** Verwendung eines monoklonalen Antikörpers nach Anspruch 11 zur Herstellung eines pharmazeutischen Mittels zur Behandlung von Tumoren.

**18.** Verwendung eines monoklonalen Antikörpers nach Anspruch 13 zur Herstellung eines pharmazeutischen Mittels zur Behandlung von Tumoren.


**Revendications**

**1.** Procédé pour la production d'un hybridome capable de produire un anticorps monoclonal appartenant à la classe des IgG comprenant

    (1) la culture d'un hybridome capable de produire un anticorps monoclonal appartenant à la classe des IgM, en présence d'un antigène réagissant avec ce dernier anticorps et en présence de thymocytes ; et
    (2) l'isolement des hybridomes produisant des IgG.

**2.** Procédé selon la revendication 1, dans lequel les hybridomes isolés sont encore cultivés, de façon optionnelle en présence de thymocytes.

**3.** Procédé selon la revendication 1, dans lequel les hybridomes isolés sont clonés par la méthode de dilution limitante.

**4.** Procédé selon l'une quelconque des revendications 1 à 3, dans lequel l'hybridome capable de produire un anticorps monoclonal appartenant à la classe des IgM est un hybridome capable de produire un anticorps monoclonal réactif vis-à-vis de N-acétyl-GM$_2$ et/ou de N-glycolyl-GM$_2$.

**5.** Procédé selon l'une quelconque des revendications 1 à 3, dans lequel l'hybridome capable de produire un anticorps monoclonal appartenant à la classe des IgM est choisi dans le groupe constitué par les hybridomes KM-531 (FERM BP-2597), KM-696 (FERM BP-3337) et KM-697 (FERM BP-3338).

**6.** Procédé selon l'une quelconque des revendications 1 à 5, dans lequel l'antigène est ajouté à la culture d'hybridome sous la forme de liposomes.

**7.** Hybridome capable de produire un anticorps monoclonal appartenant à la classe des IgG et réactif vis-à-vis de N-acétyl-GM$_2$.

**8.** Hybridome capable de produire un anticorps monoclonal appartenant à la classe des IgG et réactif vis-à-vis de N-acétyl-GM$_2$ et de N-glycolyl-GM$_2$.

**9.** Lignée cellulaire d'hybridome KM-796 (FERM BP-3340).

**10.** Lignée cellulaire d'hybridome KM-750 (FERM BP-3339).

**11.** Anticorps monoclonal appartenant à la classe des IgG et réactif vis-à-vis de N-acétyl-GM$_2$.

**12.** Anticorps monoclonal selon la revendication 11, qui est produit par la lignée cellulaire d'hybridome KM-796 (FERM BP-3340).

**13.** Anticorps monoclonal appartenant à la classe des IgG et réactif vis-à-vis de N-acétyl-GM$_2$ et de N-glycolyl-GM$_2$.

**14.** Anticorps monoclonal selon la revendication 13, qui est produit par la lignée cellulaire d'hybridome KM-750 (FERM BP-3339).

**15.** Composition pharmaceutique contenant un anticorps monoclonal appartenant à la classe des IgG et réactif vis-à-vis de N-acétyl-GM$_2$ et un support pharmaceutiquement acceptable.

**16.** Composition pharmaceutique contenant un anticorps monoclonal appartenant à la classe des IgG et réactif vis-à-vis de N-acétyl-GM$_2$ et de N-glycolyl-GM$_2$ et un support pharmaceutiquement acceptable.

**17.** Utilisation d'un anticorps monoclonal selon la revendication 11, pour la fabrication d'une composition pharmaceutique pour le traitement des tumeurs.

**18.** Utilisation d'un anticorps monoclonal selon la revendication 13, pour la fabrication d'une composition pharmaceutique pour le traitement des tumeurs.

Fig. 1

A

Sorting

B

Sorting

C    I g G peak

Sorting

Fig. 2

A — marker, I g G St, I g M St, purified monoclonal antibody KM-750

200.0 K
116.0 K
97.4 K
H (μ)
66.2 K
H (γ)
42.5 K
L

reducing condition

B — marker, I g G St, I g M St, purified monoclonal antibody KM-750

◀ 15·0 K

non-reducing condition

Fig. 3

OD415

Antibody (μg/ml)

Fig. 4

Fig. 5

CDC (IMR32/KM750)